(19) Europäisches Patentamt
European Patent Office
Office européen des brevets

(11) EP 1 934 321 B1

(12) # FASCICULE DE BREVET EUROPEEN

(45) Date de publication et mention de la délivrance du brevet:
**26.08.2009 Bulletin 2009/35**

(21) Numéro de dépôt: **06808140.5**

(22) Date de dépôt: **14.09.2006**

(51) Int Cl.:
***C11C 3/10*** *(2006.01)* ***C07C 67/03*** *(2006.01)*
***C07C 69/24*** *(2006.01)* ***C07C 69/52*** *(2006.01)*

(86) Numéro de dépôt international:
**PCT/FR2006/002117**

(87) Numéro de publication internationale:
**WO 2007/031655 (22.03.2007 Gazette 2007/12)**

(54) **PROCEDE DE FABRICATION D'ESTERS ETHYLIQUES D'ACIDES GRAS A PARTIR DE TRIGLYCERIDES ET D'ALCOOLS**

VERFAHREN ZUR HERSTELLUNG VON FETTSÄUREETHYLESTERN AUS TRIGLYCERIDEN UND ALKOHOLEN

METHOD FOR MAKING FATTY ACID ETHYL ESTERS FROM TRIGLYCERIDES AND ALCOHOLS

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **16.09.2005 FR 0509526**

(43) Date de publication de la demande:
**25.06.2008 Bulletin 2008/26**

(73) Titulaire: **Institut Français du Pétrole**
**92852 Rueil Malmaison Cedex (FR)**

(72) Inventeurs:
- **HILLION, Gérard**
  **F-95220 Herblay (FR)**
- **DELFORT, Bruno**
  **F-75005 Paris (FR)**

(56) Documents cités:
**FR-A- 2 752 242**
**FR-A- 2 866 654**
**US-A- 5 885 946**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention est relative à la fabrication d'esters éthyliques d'acides monocarboxyliques à partir d'huiles végétales ou animales.

Elle concerne plus particulièrement un nouveau procédé de fabrication d'esters éthyliques d'acides monocarboxyliques linéaires de 6 à 26 atomes de carbone, par une suite de deux étapes.

Les esters de corps gras sont actuellement utilisés dans de nombreuses applications comme carburants diesel, fuels domestiques, solvants écologiques, composés de base pour la fabrication de sulfonates d'alcools gras, d'amides, de dimères d'esters.

Dans le cas du carburant Diesel, qui constitue aujourd'hui une application majeure des esters de corps gras, un certain nombre de spécifications ont été établies dont la liste, les limites et les méthodes font partie de la norme EN 14214 (2003) applicable actuellement en Europe. L'ester doit contenir au moins 96,5 % en masse d'esters, au plus 0,8 % en masse de monoglycérides, au plus 0,2 % en masse de diglycérides et au plus 0,2 % en masse de triglycérides, peu d'acides gras libres, qui peuvent être corrosifs, moins de 0,25 % en masse de glycérine liée et libre et pas du tout d'acides forts ou de traces de métaux. Ceci implique un protocole précis pour obtenir la pureté désirée.

Dans le cas du fuel domestique, il est évident que toutes ces spécifications ne sont pas toujours utiles et sont même parfois préjudiciables mais le marché du fuel domestique et celui du gazole étant souvent confondus, les spécifications du fuel domestique ressemblent à celle du gazole, car en France on peut utiliser le fuel domestique dans les tracteurs agricoles et des engins de chantiers.

Lorsqu'on fabrique un ester à partir d'huile ou de graisse et de monoalcool, il se forme automatiquement, selon la nature de l'huile engagée au départ, de 10 à 15 % en masse d'un produit secondaire, qui est la glycérine. Cette glycérine est vendue à un prix élevé pour des utilisations variées, mais seulement lorsqu'elle possède une grande pureté. Celle-ci est obtenue après des purifications poussées dans des unités spécialisées dans la distillation sous vide.

En résumé, la plupart des procédés commerciaux de fabrication d'esters aboutissent assez facilement à des produits bruts (esters et glycérine), qu'il faut cependant purifier de façon approfondie par divers traitements qui grèvent finalement le prix de la transformation.

Aussi, dans la fabrication d'esters méthyliques de corps gras à partir d'huiles raffinées et d'alcool sec, alors qu'on utilise couramment comme catalyseurs des dérivés alcalins simples, comme les alcoolates de sodium, la soude ou la potasse, dans des conditions assez douces (température de 50 à 80°C et pression atmosphérique), ainsi qu'on peut le lire dans de nombreux brevets ou publications, par exemple dans le JAOCS 61, 343-348 (1984), on n'arrive cependant à un produit pur utilisable comme carburant et une glycérine aux normes qu'après de très nombreuses étapes.

Si l'on prend par exemple les catalyseurs alcalins les plus utilisés, on retrouve, aussi bien dans la glycérine que dans l'ester, des traces de composés alcalins, qu'il faut éliminer par lavage de la fraction ester et séchage de celle-ci. Dans la phase glycérine, il faut neutraliser les savons et les alcoolates présents, filtrer les sels formés, évaporer la glycérine après avoir éliminé l'eau, à moins que l'on passe la glycérine diluée sur des résines échangeuses d'ions, avant de concentrer la glycérine exempte de sels. Enfin, il faut toujours évaporer l'alcool en excès et souvent le distiller, en évitant que cette évaporation, surtout lorsqu'elle est réalisée dans la phase ester, ne conduise à faire réagir l'ester présent avec la glycérine dissoute partiellement, ce qui conduirait à la formation de monoglycérides.

En résumé, pour obtenir les spécifications recherchées pour la glycérine et l'ester, il est nécessaire de recourir à de si nombreuses étapes que seules de grandes unités sont capables, dans ces conditions, d'être économiquement rentables.

Par ailleurs, si les esters d'huiles végétales ou animales à usage carburant diesel sont le plus souvent des esters méthyliques, on peut tout aussi bien utiliser des esters éthyliques d'huiles végétales ou animales. La fabrication de ces derniers se fait à partir de l'éthanol au lieu du méthanol, ce qui conduit lorsque l'éthanol est d'origine renouvelable à un carburant biodiesel dont l'origine est 100 % renouvelable, ce qui n'est pas le cas des esters méthyliques d'acides gras, le méthanol étant généralement obtenu à partir de matière fossile.

La fabrication d'esters éthyliques d'acides gras peut être aussi préférée dans les zones géographiques où l'éthanol est plus abondant ou plus disponible que le méthanol.

On a maintenant découvert de façon surprenante que l'on pouvait obtenir à partir d'huiles végétales ou animales d'une façon économique un mélange d'esters d'acides gras riches en esters éthyliques d'acides gras et une glycérine de grande pureté en opérant en deux étapes en utilisant du méthanol lors de la première étape et de l'éthanol lors de la seconde.

Ainsi, l'invention propose un procédé de fabrication d'esters éthyliques d'acides monocarboxyliques linéaires de 6 à 26 atomes de carbone, par réaction d'huiles végétales ou animales, neutres ou acides, vierges ou recyclées, en présence d'un catalyseur hétérogène comprenant une combinaison formée entre au moins un oxyde métallique et de l'alumine ou une combinaison formée entre au moins deux oxydes métalliques et éventuellement avec de l'alumine, ledit procédé étant caractérisé en ce qu'il associe deux étapes de transestérification successives, la première utilisant du méthanol et la seconde de l'éthanol.

Les deux étapes du procédé de l'invention sont décrites plus particulièrement ci-après :

Étape 1

Elle comprend la réaction d'au moins une huile végétale ou animale, neutre ou acide, vierge ou recyclée, avec du méthanol, en présence d'un catalyseur hétérogène comprenant une combinaison formée entre au moins un oxyde métallique et de l'alumine ou une combinaison formée entre au moins deux oxydes métalliques et éventuellement avec de l'alumine.

La réaction qui est visée de façon principale dans cette étape est une transestérification réalisée selon le Schéma I ci-dessous, illustrée ici avec des chaînes grasses oléiques.

Schéma I

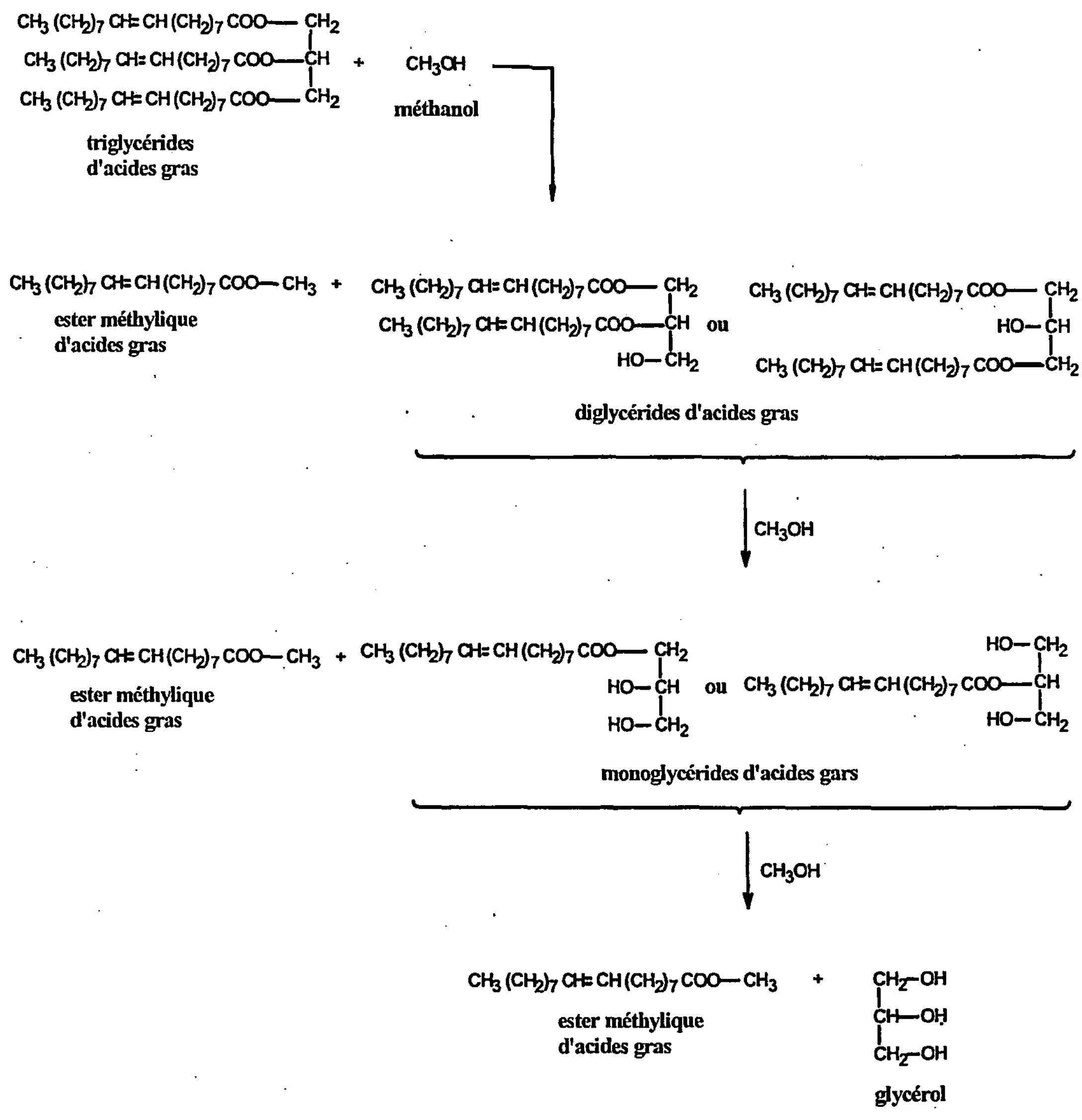


Le produit obtenu contient majoritairement des esters méthyliques d'acides gras, mais contient également des monoglycérides et des diglycérides d'acides gras, qui sont des produits intermédiaires de la transformation, ainsi que des triglycérides d'acides gras résiduels.

Étape 2

Elle comprend la réaction du produit issu de l'étape 1 avec de l'éthanol en présence d'un catalyseur hétérogène défini de la même manière que le catalyseur hétérogène utilisé dans l'étape 1.

La transformation réalisée lors de cette étape conduit à des compositions d'esters éthyliques et d'esters méthyliques d'acides gras, riches en esters éthyliques et dont les concentrations en monoglycérides, en diglycérides d'acides gras et en triglycérides d'acides gras résiduels sont compatibles avec les spécifications requises pour que les produits soient utilisables comme carburants ou co-carburants pour moteur diesel.

Les réactions qui sont visées de façon principale dans l'étape 2 sont des transestérifications, réalisées selon le Schéma II ci-dessous, illustrées ici avec des chaînes grasses oléiques.

Schéma II

$$CH_3(CH_2)_7CH{=}CH(CH_2)_7COO{-}CH_3 + CH_3CH_2OH \longrightarrow CH_3(CH_2)_7CH{=}CH(CH_2)_7COO{-}CH_2CH_3 + CH_3OH$$

ester méthylique d'acides gras — éthanol — esters éthyliques d'acides gras — méthanol

mais aussi selon le schéma :

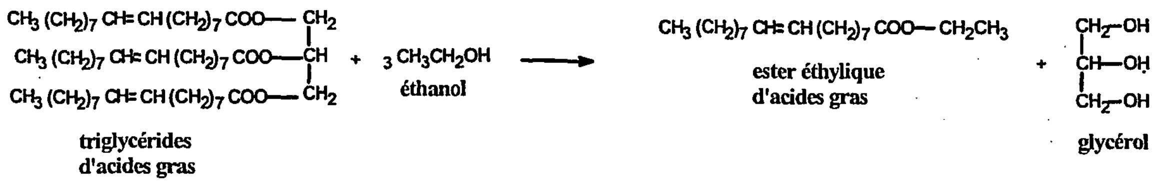


et

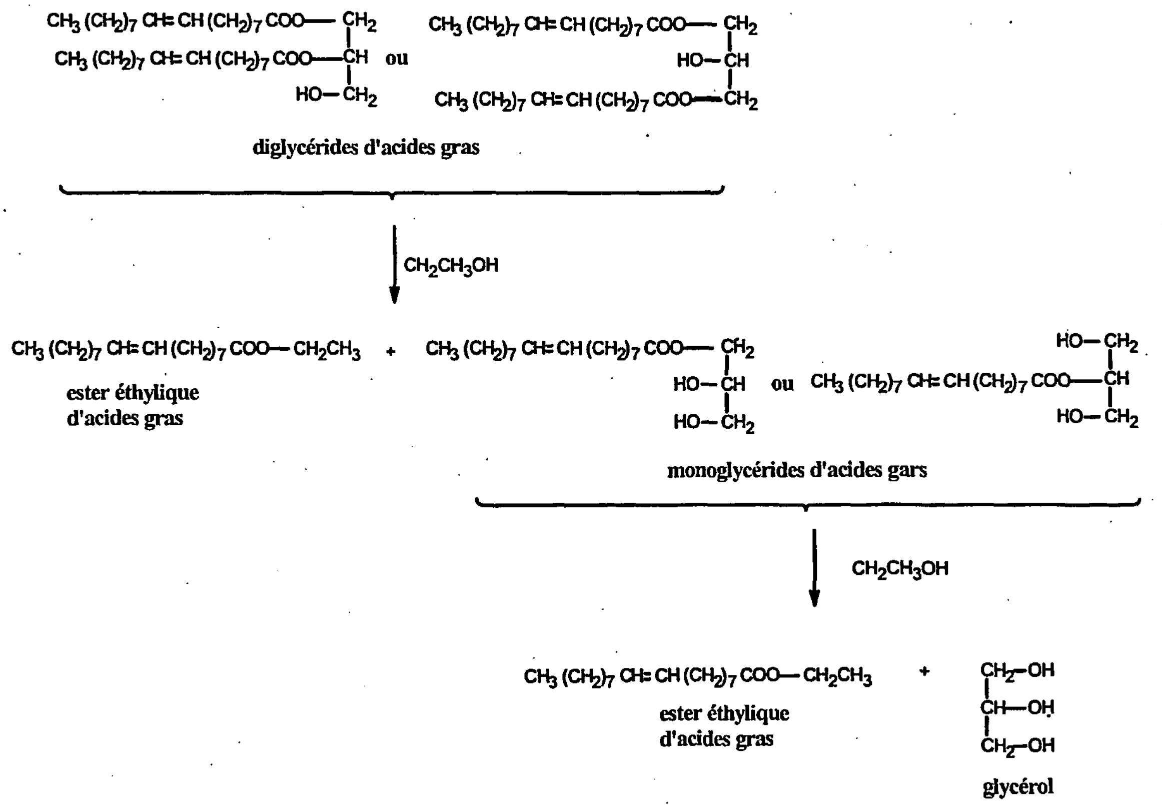


Dans les deux étapes de transestérification du procédé de l'invention, par catalyseur hétérogène, on entend tout catalyseur solide permettant de transestérifier un mélange de triglycérides tel qu'un corps gras (huile ou graisse) naturel en un mélange d'esters alkyliques. Le catalyseur hétérogène peut être choisi plus particulièrement parmi les oxydes métalliques et les mélanges d'oxydes susceptibles de former entre eux, et éventuellement avec de l'alumine, des combinaisons d'oxydes.

L'utilisation de catalyseurs hétérogènes n'est pas nouvelle. Toutefois, dans aucun procédé industriel, il ne semble que l'on puisse obtenir de façon économique à la fois un ester et une glycérine de grande pureté. Produire économiquement signifie obtenir un ester aux normes et une glycérine pure, à une bonne vitesse spatiale ou dans des temps corrects et sans épuration fastidieuse.

Parmi les documents antérieurs qui traitent de catalyseurs hétérogènes, on peut citer le brevet européen EP-B-0 198 243. Le catalyseur de transestérification, qui transforme huile et méthanol en ester méthylique, est une alumine ou un mélange d'alumine et d'oxyde ferreux. Dans les exemples, la colonne utilisée pour le lit fixe à un volume de 10 litres et l'on injecte généralement de l'huile à un débit inférieur à 1 litre/heure, ce qui donne une WH (VVH = volume d'huile injecté/volume de catalyseur/heure) inférieure à 0,1. Pour une usine de 100 000 t/an, cela correspondrait à des réacteurs d'au moins 150 $m^3$. Un autre problème qui semble se poser est celui de la quantité de glycérine recueillie, très inférieure à la théorie. Dans aucun exemple où l'on est censé recueillir 10 % en masse de glycérine, on n'obtient, même de manière approchée, cette valeur. Enfin, la pureté des esters est assez faible, de 93,5 à 98 %. Ce que devient la glycérine qui n'est pas récupérée n'est pas indiqué. Dans certains cas, il se forme des éthers de glycérine, comme cela est signalé dans ce brevet ; dans d'autres cas, peut-être se décompose-t-elle, à moins qu'elle ne soit éliminée dans une première étape. Le niveau de performance est donc assez bas. On peut signaler qu'aux WH indiquées et pour des

temps de contact de plus de 6 heures, on peut obtenir même sans catalyseur des conversions de 80 % et plus.

Ce brevet ne semble donc pas présenter une solution raisonnable du point de vue économique.

La demande de brevet britannique GB-A-795 573 décrit l'utilisation comme catalyseur d'un silicate de zinc à des températures comprises entre 250 et 280°C et sous une pression d'au moins 100 bar (10 MPa), avec le méthanol. Il semble qu'il y ait dans une première étape 85 % de conversion et 100 % si l'on décante la glycérine de façon intermédiaire et que l'on continue la réaction. Selon le brevet EP-B-0 198 243, qui cite GB-A-795 573, il se formerait avec les composés au zinc des savons de zinc, qui sont naturellement à proscrire dans des fuels. Ceci est surtout dû, semble-t-il, aux températures élevées qu'il est nécessaire de mettre en oeuvre dans cette réaction avec ce catalyseur. Depuis, une nouvelle génération de catalyseurs hétérogènes permettent d'obtenir à partir d'huiles végétales ou animales et de monoalcools, des esters d'une grande pureté et répondants aux spécifications de la norme EN 14214 et une glycérine incolore et parfois inodore selon le post traitement utilisé, décoloration sur charbon, terre activée, autres adsorbants). Les procédés utilisés avec ces catalyseurs hétérogènes fonctionnent, soit en continu, par exemple en lit fixe, soit en discontinu. Les catalyseurs sont constitués d'un système catalytique à base d'oxydes métalliques seuls ou associés, déposés ou non sur une alumine.

On peut citer les brevets de la demanderesse qui mettent en oeuvre des catalyseurs à base d'oxydes métalliques et d'alumine : notamment le brevet FR-B-2 752 242, qui décrit l'utilisation de catalyseurs solides et non solubles formés à partir d'oxyde de zinc et d'alumine ou d'aluminate de zinc, le brevet français FR-B-2 838 433, les demandes de brevets français publiées FR-A-2 855 517, 2 855 518, 2 855 519, 2 869 612 et 2 869 613.

Tous ces catalyseurs sont sous forme de poudres, de billes, d'extrudés ou de pastilles. Toutefois, l'association d'alumine dans les systèmes catalytiques a deux effets favorables. Le premier est d'augmenter souvent la surface, le second est de créer un composé beaucoup plus stable, surtout vis-à-vis de conditions dans lesquelles le métal composant l'oxyde aurait tendance à former des savons métalliques.

Un autre intérêt des catalyseurs à base d'oxydes est leur capacité à catalyser la transestérification de l'huile avec des alcools plus lourds que le méthanol. Ainsi, on peut former des esters éthyliques, isopropyliques ou butyliques, qui ont un intérêt dans le domaine des carburants, car souvent les points d'écoulement des esters formés avec ces alcools sont plus bas que ceux des esters méthyliques, le gain étant parfois de 10°C, ce qui permet d'utiliser au départ des huiles plus saturées.

Parmi les oxydes métalliques utilisables dans les combinaisons d'oxydes, on trouve, outre l'alumine, les oxydes des métaux du groupe IIA comme le magnésium, le calcium, le strontium et le baryum, du groupe IIB comme le zinc et le cadmium, du groupe IIIA comme le gallium et l'indium, du groupe IIIB comme le scandium, l'yttrium, le lanthane, l'actinium, le cérium et le thorium, du groupe IVB comme le titane, te zirconium et l'hafnium, du groupe IIIA comme le gallium, l'indium et le thallium, du groupe IVA comme le germanium, l'étain et le plomb, et le groupe VA comme l'arsenic, l'antimoine et le bismuth.

Leur formules générales, pour une association d'un seul oxyde avec de l'alumine, est la suivante : $(M^1O_x)_y$ $(Al_2O_3)_{(1-y)}$, (x ayant une valeur de 1,2 à 2,6 et y représentant le rapport massique des deux oxydes ayant une valeur de 0,005 à 2).

Pour une association de deux oxydes de métaux $M^1$ et $M^2$ entre eux, éventuellement en présence d'alumine, on a la formule générale

$$[M^1{}_aM^2{}_b\,O_c]_y\,[(Al_2O_3)]_{(1-y)}$$

(où a a une valeur de 0,5 à 5, <u>b</u> une valeur de 0,5 à 5, <u>c</u> est le nombre d'atomes d'oxygène qui satisfait la valence des métaux $M^1$ et $M^2$ et y a une valeur de 0,005 à 1).

Les deux étapes du procédé de l'invention sont réalisées dans des conditions opératoires classiques, par exemple à une température de 165 à 240°C, avec un temps de contact de 15 minutes à 3 heures et un rapport massique alcool/charge de 20/80 à 80/20.

On a constaté que l'enchaînement de ces deux étapes, la première avec du méthanol et la seconde avec de l'éthanol, selon le procédé de l'invention, présente certains avantages par rapport à un procédé hétérogène classique (tel que décrit par exemple dans le brevet US-A-5 908 946 de la demanderesse, ou FR-B-2 752 242 cité plus haut), dans lequel, pour obtenir des esters éthyliques d'acides gras, les deux étapes seraient effectuées avec de l'éthanol.

Parmi les huiles utilisables dans le procédé de l'invention, on peut citer toutes les huiles courantes, comme les huiles de palme (concrètes ou oléines), de soja, de palmiste, de coprah, de babassu, de colza (ancien ou nouveau), de tournesol (classique ou oléique), de maïs, de coton, le suif et le saindoux, les huiles d'arachide, de pourghère (*Jatropha curcas*), de ricin, de lin et de crambe et toutes les huiles issues par exemple du tournesol ou du colza par modification génétique ou hybridation.

On peut même utiliser des huiles de friture, d'équarrissage, des huiles animales variées, comme les huiles de poissons, de phoques, et même des graisses de volailles, car les esters fabriqués à partir de certains alcools, permettent de gagner plus de 10°C en point d'écoulement.

Parmi les huiles utilisées, on peut encore indiquer des huiles partiellement modifiées par exemple par polymérisation ou oligomérisation.

La présence d'acide gras dans les huiles n'est pas a priori préjudiciable car les systèmes catalytiques à base d'oxydes sont également actifs pour l'estérification et transforment également les acides gras en esters. La valeur limite en acides gras libres contenue dans les huiles se situent à un indice d'acide voisin de 10. L'opérabilité du procédé dans ces conditions est proche de celle définie avec une huile à faible indice d'acide.

Dans le cas d'huiles à très fort indice d'acide une possibilité est de faire précéder la réaction de transestérification d'une réaction d'estérification des acides gras libres présents, soit en utilisant le même alcool que celui utilisé dans le procédé de transestérification en présence d'un acide fort comme l'acide sulfurique ou des acides sulfoniques solubles ou supportés (de type résines Amberlyst 15®), soit en utilisant de préférence de la glycérine, pour former un ester de glycérol total ou partiel, en utilisant le même type de catalyseur à base d'oxydes métalliques, à pression atmosphérique et de préférence sous un vide partiel et à des températures comprises entre 180 et 240°C.

Avec les huiles de friture, qui sont une matière première très bon marché pour produire un biodiesel, ce sont les polymères d'acides gras qui devront être éliminés du mélange réactionnel afin que le mélange d'esters réponde aux spécifications de la norme EN 14214.

**EXEMPLES**

Les exemples suivants illustrent l'invention, les Exemples 1, 3 et 5 étant donnés à titre de comparaison.

L'huile utilisée dans ces exemples est de l'huile de colza dont la composition en acides gras est la suivante :

| Glycéride d'acides gras | Nature de la chaîne grasse | % en masse |
|---|---|---|
| Palmitique | C16 :0 | 5 |
| Palmitoléique | C16 :1 | < 0,5 |
| Stéarique | C18 :0 | 2 |
| Oléique | C18 :1 | 59 |
| Linoléique | C18 :2 | 21 |
| Linoléique | C18 :3 | 9 |
| Arachidique | C20 :0 | < 0,5 |
| Gadoléique | C20 :1 | 1 |
| Béhénique | C22 :0 | < 0,5 |
| Erucique | C22 :1 | < 1 |

Cependant, toute autre huile d'origine végétale ou animale pourrait donner des résultats analogues.

**<u>Exemple 1 (comparatif)</u>**

L'Exemple 1 détaille les conditions opératoires d'un procédé hétérogène utilisant deux étapes de transestérification dans lequel l'alcool mis en jeu est l'éthanol. Ces deux étapes sont nécessaires pour atteindre le degré de pureté demandé à un mélange d'esters à usage carburant, selon la norme EN 14214. Le schéma de principe est donné à la Figure 1.

<u>Description du dispositif expérimental</u>

On réalise l'expérience dans un appareil comportant un réacteur à lit fixe, c'est-à-dire une colonne remplie, de diamètre égal à 1,9 cm et de longueur égale à 120 cm, chauffée par trois coquilles qui entourent la colonne. Le préchauffage de l'huile et de l'alcool se fait dans la colonne sur 10 $cm^3$ de billes de verre et la réaction sur 110 $cm^3$ de volume d'un catalyseur solide constitué majoritairement d'aluminate de zinc sous la forme d'extrudés d'environ 2 mm de diamètre, répondant à la formule : $ZnAl_2O_4$, x ZnO, y $Al_2O_3$ (x et y étant compris chacun entre 0 et 2) et préparé selon un protocole décrit dans le brevet FR-B-2 752 242. À la sortie de la colonne, on a rajouté 20 cm de carbure de tungstène et 5 $cm^3$ de billes de verre. Le dispositif, en U renversé, est constitué d'un réacteur tubulaire, d'un refroidissement sur la partie horizontale et d'un décanteur, qui constitue la deuxième branche. Sur la partie supérieure du

décanteur, un système de purge gazeuse permet de réguler la pression, c'est-à-dire de maintenir celle-ci au départ avec de l'azote à la pression désirée de 15 à 60 bar (1-6 MPa). Le décanteur possède à sa sortie inférieure une purge liquide. Lorsque le décanteur est à moitié plein, une vanne automatique s'ouvre pour vider partiellement le produit obtenu. Deux pompes injectent aux débits choisis et à pression constante l'alcool et l'huile dans la colonne et de bas en haut.

Après avoir soutiré le produit constitué d'alcool, de glycérol et d'ester, généralement présents en une seule phase, on évapore l'alcool, puis on sépare l'ester et le glycérol par décantation. L'analyse de la phase ester se fait par chromatographie d'exclusion stérique. La composition du mélange est exprimée en % poids. La WH est le volume d'huile injecté par volume de catalyseur et par heure. Le temps de séjour tient compte de la présence d'alcool ; il est déterminé par la relation :

$$\frac{120\ cm^3\ \text{de catalyseur x } 60(*)}{\text{volume d'huile en } cm^3 + \text{volume d'alcool en } cm^3\ \text{(injectés en 1 heure)}}$$

(*) le temps est exprimé en minutes.

<u>Expérimentation</u>

Dans un réacteur à lit fixe contenant 110 ml d'un catalyseur constitué d'un oxyde mixte aluminate de zinc, on introduit à 200°C 100 g d'huile de colza et 100 g d'éthanol, à une VVH de 0,25 $h^{-1}$ (volume d'huile par volume de catalyseur et par heure).

A l'issue d'un temps de séjour d'environ 110 minutes, on obtient un mélange constitué d'environ 93,2 % en masse d'esters éthyliques, de 3,7 % en masse de monoglycérides, le complément à 100 % étant constitué de triglycérides, de diglycérides, de stérols et d'esters de stérols. Cette composition n'est pas compatible avec les spécifications requises par la norme EN 14214 pour utiliser ce produit comme biocarburant, au moins en ce qui concerne les proportions d'esters et de monoglycérides. Aussi convient-il de poursuivre la réaction afin d'améliorer la conversion.

Pour réaliser une deuxième étape de catalyse, il est nécessaire d'éliminer tout ou partie de la glycérine formée au cours de la première étape afin de déplacer l'équilibre de la réaction. Un flash de l'excès d'éthanol est facilement obtenu par détente en passant de 200°C à 90°C, où au moins 75 % de l'éthanol présent est éliminé. Dans ces conditions, une grande partie de la glycérine est éliminée par décantation. La phase ester est alors introduite avec un complément d'éthanol dans un second réacteur identique au premier, pour y subir un complément de conversion.

Durant cette deuxième étape, la température, le temps de séjour et la quantité d'éthanol sont ajustés pour satisfaire les spécifications requises pour que le produit soit utilisable comme biodiesel selon la norme EN 14214 : au moins 96,5 % en masse d'esters, au plus 0,8 % en masse de monoglycérides, au plus 0,2 % en masse de diglycérides et au plus 0,2 % en masse de triglycérides. Pour un rapport massique éthanol/phase ester de 1/1, à 200°C, le temps de réaction nécessaire pour atteindre la conversion permettant de satisfaire les spécifications exigée pour le biodiesel est de 110 minutes.

A ce stade, l'excès d'éthanol contenu dans le mélange d'ester ainsi obtenu doit être de nouveau éliminé par évaporation. Une seconde fraction de glycérine, qui représente environ 10 à 12 % de la glycérine totale formée au cours de la réaction de transestérification, peut être alors éliminée par décantation. L'éthanol résiduel est alors totalement éliminé par distillation, ce qui constitue l'ultime étape de purification de la fraction d'esters. Le rendement en biodiesel est proche du maximum théorique, car dans ce cas, les composés insaponifiables naturellement présents dans l'huile sont contenus dans le biodiesel.

**<u>Exemple 2 (selon l'invention)</u>**

Cet exemple décrit les conditions opératoires du procédé mettant en oeuvre une première étape de transestérification dans laquelle l'alcool mis en jeu est le méthanol et une deuxième étape dans laquelle l'alcool mis en jeu est l'éthanol. Le schéma de principe est donné à la Figure 2.

Comme dans l'Exemple 1, dans un réacteur à lit fixe contenant 110 ml du même catalyseur, on introduit à 200°C 100 g d'huile de colza et cette fois 100 g de méthanol, à une VVH de 0,5 $h^{-1}$ (volume d'huile par volume de catalyseur et par heure), soit un temps de réaction de 55 minutes. La composition du mélange réactionnel à l'issue de ce temps de réaction est de l'ordre de 93,9 % en masse d'esters méthyliques, de 3,4 % en masse de monoglycérides et le complément à 100 % étant constitué de triglycérides résiduels, de diglycérides, de stérols et d'esters de stérols.

L'étape suivante consiste à éliminer la totalité de l'excès de méthanol, à décanter la glycérine formée, puis à poursuivre l'opération par une deuxième étape de réaction au cours de laquelle la phase ester est introduite avec de

l'éthanol dans un second réacteur identique au premier pour être soumise à une transformation en esters éthyliques d'acides gras.

Durant cette deuxième étape de réaction, la température, le temps de séjour, et la quantité d'éthanol sont ajustés pour satisfaire les spécifications requises pour que le produit soit utilisable comme biodiesel : au moins 96,5 % en masse d'esters, au plus 0,8 % en masse de monoglycérides, au plus 0,2 % en masse de diglycérides et au plus 0,2 % en masse de triglycérides. Pour un rapport massique éthanol/phase ester de 1/1, à 200°C le temps de réaction nécessaire pour atteindre la conversion permettant de satisfaire les spécifications exigée pour le biodiesel est de 55 minutes.

L'alcool résiduel contenu dans le mélange d'ester ainsi obtenu doit être de nouveau éliminé par évaporation. Il s'agit d'un mélange d'éthanol et de méthanol, riche en éthanol. Les deux alcools seront séparés par distillation selon des procédures bien connues à la disposition de l'homme de l'art, puis recyclés dans le procédé. Une seconde fraction de glycérine qui représente environ 10 à 12 % de la glycérine totale formée au cours de la réaction de transestérification, peut être alors éliminée par décantation. Le rendement en biodiesel est proche du maximum théorique, car dans ce cas, les composés insaponifiables naturellement présents dans l'huile sont contenus dans le biodiesel.

Le biodiesel obtenu est constitué de 97,5 % d'un mélange d'ester éthylique d'acides gras et d'esters méthyliques d'acides gras dont la proportion massique esters éthyliques/esters méthyliques est 72/28.

Ainsi, en opérant de la sorte, on a pu préparer un biocarburant composé d'esters éthyliques d'acides gras et d'esters méthyliques d'acides gras, riche en esters éthylique d'acides gras, et ce avec des temps de réactions dans chacune des deux étapes de 55 minutes contre 110 minutes lorsque les deux étapes sont réalisées chacune en présence d'éthanol, comme décrit dans l'Exemple 1.

**<u>Exemple 3 (comparatif)</u>**

Dans un réacteur autoclave de 100 ml équipé d'un système d'agitation et d'un contrôle de température et de pression, on introduit 25 g d'huile de colza, 25 g d'éthanol et 1 g du catalyseur décrit dans l'Exemple 1. Le milieu est porté à 200 °C sous agitation. La pression atteint 32 bar (3,2 MPa).

Un échantillon est prélevé dans la phase liquide après 7 heures de réaction. Après filtration et évaporation de l'éthanol, le milieu est laissé à décanter. Aucune séparation de phase n'est observée, ce qui indique que la formation de glycérol libre est négligeable. La concentration en esters éthyliques est déterminée par chromatographie d'exclusion stérique. Elle est de 23,8 % en masse.

A ce stade, dans un réacteur autoclave de 100 ml équipé d'un système d'agitation et d'un contrôle de température et de pression, on introduit 20 g du produit obtenu précédemment, 20 g d'éthanol et 1 g de catalyseur décrit dans l'Exemple 1. Le milieu est porté à 200 °C sous agitation. La pression atteint 32 bar (3,2 MPa).

Un échantillon est prélevé dans la phase liquide après 7 heures de réaction. Après filtration et évaporation de l'éthanol en excès puis élimination du glycérol formé par décantation, la concentration en esters éthyliques est déterminée par chromatographie d'exclusion stérique. Elle est de 41,2 % en masse. Cette conversion exclut l'utilisation du produit obtenu comme biodiesel.

**<u>Exemple 4 (selon l'invention)</u>**

Dans un réacteur autoclave de 100 ml équipé d'un système d'agitation et d'un contrôle de température et de pression, on introduit 25 g d'huile de colza, 25 g de méthanol 1 g du catalyseur décrit dans l'Exemple 1. Le milieu est porté à 200 °C sous agitation. La pression atteint 32 bar (3,2 MPa).

Un échantillon est prélevé dans la phase liquide après 7 heures de réaction. Après filtration et évaporation du méthanol en excès puis élimination du glycérol, formé par décantation, la concentration en esters, déterminée par chromatographie d'exclusion stérique est de 94,9 % en masse d'esters, 3,4% en masse de monoglycérides, le complément à 100 % étant constitué de diglycérides, de triglycérides résiduels, de stérols et d'esters de stérols.

A ce stade, dans un réacteur autoclave de 100 ml équipé d'un système d'agitation et d'un contrôle de température et de pression, on introduit 20 g du produit précédemment, 20 g d'éthanol et 1 g de catalyseur décrit dans l'Exemple 1. Le milieu est porté à 200 °C sous agitation. La pression atteint 32 bar (3,2 MPa).

Un échantillon est prélevé dans la phase liquide après 7 heures de réaction. Après filtration et évaporation des alcools en excès puis élimination du glycérol formé par décantation, la concentration en esters, déterminée par chromatographie d'exclusion stérique est de 96,9% en masse d'esters, 0,7 % en masse de monoglycérides, 0,15 % en masse de diglycérides et 0,1 % en masse de triglycérides. Le complément à 100 % étant constitué de stérols et d'esters de stérols. La proportion massique d'esters éthyliques/esters méthyliques est ici 64/36. Cette composition est compatible avec l'utilisation comme biocarburant dans le moteur diesel.

Ainsi, en opérant de la sorte, on a pu préparer un biocarburant composé d'esters éthylique d'acides gras et d'esters méthyliques d'acides gras, riche en esters éthyliques d'acides gras, contrairement à ce qui est obtenu dans

l'Exemple 3 lorsque les deux étapes sont réalisée chacune en présence d'éthanol.

**Exemple 5 (comparatif)**

Cet exemple décrit les conditions opératoires du procédé mettant en oeuvre deux étapes de transestérification utilisant chacun un mélange méthanol / éthanol à 50/50 en poids.

Comme dans l'Exemple 1, dans un réacteur à lit fixe contenant 110 ml du même catalyseur, on introduit à 200°C, 100 g d'huile de colza et cette fois 100 g d'un mélange 50/50 poids de méthanol et d'éthanol, à une V.V.H. de 0,5 $h^{-1}$ (volume d'huile par volume de catalyseur et par heure), soit un temps de réaction de 55 minutes.

La composition du mélange réactionnel à l'issue de ce temps de réaction est de l'ordre de 92,7% en masse d'un mélange d'esters méthyliques et éthyliques, le complément à 100% étant constitué de monoglycérides, diglycérides, triglycérides, de stérols et d'esters de stérols.

L'étape suivante consiste à éliminer du milieu réactionnel la totalité des alcools en excès, et d'éliminer après décantation la glycérine formée, avant d'effectuer une deuxième étape de transestérification dans des conditions identiques à la première. Pour 100g de mélange d'esters ainsi obtenus, on introduit 100 g du même mélange 50/50 poids méthanol / éthanol, à une température de 200°C et à une V.V.H. de 0,5 $h^{-1}$, ce qui correspond à un temps de réaction de 55 minutes.

Après élimination totale de l'excès d'alcool par distillation et élimination de la glycérine formée, le mélange obtenu est constitué de 97,1 % en masse d'un mélange d'esters d'acides gras dont la proportion massique en esters éthyliques et esters méthyliques est 21/79.

En opérant de la sorte et où les quantités respectives en méthanol et en éthanol de l'exemple 2 sur les 2 étapes ont été conservées, la participation de l'éthanol comme biocarburant est largement minoritaire par rapport au méthanol, ce qui va à l'encontre du résultat obtenu selon l'invention dans l'exemple 2.

## Revendications

1. Procédé de fabrication d'une composition d'esters comprenant des esters éthyliques d'acides monocarboxyliques linéaires de 6 à 26 atomes de carbone à partir d'une huile végétale ou animale, neutre ou acide, vierge ou recyclée, ledit procédé étant **caractérisé en ce qu'**il comprend :

   - une étape 1 de réaction de ladite huile végétale ou animale, avec du méthanol, en présence d'un catalyseur hétérogène comprenant une combinaison formée entre au moins un oxyde métallique et de l'alumine ou une combinaison formée entre au moins deux oxydes métalliques, et
   - une étape 2 de réaction du produit issu de l'étape 1, avec de l'éthanol en présence d'un catalyseur hétérogène défini de la même manière que le catalyseur hétérogène utilisé dans l'étape 1.

2. Procédé de fabrication selon la revendication 1 dans lequel ledit catalyseur comprend une combinaison formée entre au moins deux oxydes métalliques avec de l'alumine.

3. Procédé selon l'une des revendications 1 ou 2 **caractérisé en ce que** chacune des étapes 1 et 2 est réalisée à une température de 165 à 240°C, avec un temps de contact de 15 minutes à 3 heures et un rapport massique alcool/charge de 20/80 à 80/20.

4. Procédé selon l'une des revendications 1 à 3 **caractérisé en ce que** l'huile de départ est choisie parmi les huiles de palme (concrètes ou oléines), de soja, de palmiste, de coprah, de babassu, de colza ancien ou nouveau, de tournesol classique ou oléique, de maïs, de coton, le suif et le saindoux, les huiles d'arachide, de pourghère, de ricin, de lin et de crambe et les huiles du tournesol ou du colza obtenus par modification génétique ou par hybridation.

5. Procédé selon l'une des revendications 1 à 3 **caractérisé en ce que** l'huile de départ est choisie parmi les huiles de friture, d'équarrissage, les huiles de poissons, de phoques, les graisses de volailles.

6. Procédé selon l'une des revendications 1 à 3 **caractérisé en ce que** l'huile de départ est choisie parmi les huiles partiellement modifiées par polymérisation ou oligomérisation.

7. Procédé selon l'une des revendications 1 à 3 **caractérisé en ce que** l'huile de départ a un indice d'acide voisin de 10.

8. Procédé selon l'une des revendications 1 à 3 **caractérisé en ce que**, l'huile de départ ayant un indice d'acide

supérieur à 10, l'on fait précéder la réaction de transestérification d'une réaction d'estérification des acides gras libres présents au moyen du même alcool que celui utilisé dans le procédé de transestérification en présence d'un acide fort choisi parmi l'acide sulfurique, les acides sulfoniques solubles ou supportés.

**9.** Procédé selon l'une des revendications 1 à 3 **caractérisé en ce que**, l'huile de départ ayant un indice d'acide supérieur à 10, l'on fait précéder la réaction de transestérification d'une réaction d'estérification des acides gras libres présents au moyen de glycérine pour former un ester de glycérol total ou partiel, en utilisant un catalyseur hétérogène, à pression atmosphérique ou sous un vide partiel et à une température comprise entre 165 et 240°C.

**10.** Procédé selon l'une des revendications 1 à 9 **caractérisé en ce que** ledit catalyseur hétérogène mis en jeu dans l'étape 1 ou ledit catalyseur hétérogène mis en jeu dans l'étape 2 comprend :

- une combinaison d'au moins un oxyde d'au moins un métal $M^1$ choisi les métaux du groupe IIA, du groupe IIB, du groupe IIIA, du groupe IIIB, du groupe IVB, du groupe IVA et du groupe VA avec de l'alumine ;
- ou une combinaison d'au moins deux oxydes d'au moins un métal $M^1$ et d'au moins un métal $M^2$ choisis parmi les métaux du groupe IIA, du groupe IIB, du groupe IIIA, du groupe IIIB, du groupe IVB, du groupe IVA et du groupe VA .

**11.** Procédé selon la revendication 10 dans lequel ledit catalyseur comprend une combinaison d'au moins deux oxydes d'au moins un métal $M^1$ et d'au moins un métal $M^2$ choisis parmi les métaux du groupe IIA, du groupe IIB, du groupe IIIA, du groupe IIIB, du groupe IVB, du groupe IIIA, du groupe IVA et du groupe VA en présence d'alumine

**12.** Procédé selon la revendication 10 **caractérisé en ce que** ledit catalyseur hétérogène comprend une combinaison d'oxydes répondant à la formule générale : $(M^1O_x)_y\ (Al_2O_3)_{(1-y)}$, où $\underline{x}$ a une valeur de 1,2 à 2,6 et y, représentant le rapport massique des deux oxydes, a une valeur de 0,005 à 2.

**13.** Procédé selon la revendication 10 **caractérisé en ce que** ledit catalyseur hétérogène comprend une combinaison d'oxydes répondant à la formule générale :

$$[M^1_aM^2_bO_c]_y\ [(Al_2O_3)]_{(1-y)}$$

où $\underline{a}$ a une valeur de 0,5 à 5, $\underline{b}$ une valeur de 0,5 à 5, $\underline{c}$ est le nombre d'atomes d'oxygène qui satisfait la valence des métaux $M^1$ et $M^2$ et y a une valeur de 0,005 à 1.

## Claims

**1.** A method of manufacturing an ester composition comprising ethyl esters of linear monocarboxylic acids having 6 to 26 carbon atoms from a vegetable or an animal oil, neutral or acid, virgin or recycled, said method being **characterized in that** it comprises:

- a stage 1 of reaction of said vegetable or animal oil with methanol, in the presence of a heterogeneous catalyst comprising a combination of at least one metallic oxide and alumina or a combination of at least two metallic oxides and possibly alumina, and
- a stage 2 of reaction of the product from stage 1 with ethanol in the presence of a heterogeneous catalyst defined in the same manner as the heterogeneous catalyst used in stage 1.

**2.** A method as claimed in claim 1 wherein said catalyst comprises a combination of at least two metallic oxides with alumina.

**3.** A method as claimed in any one of claims 1 or 2, **characterized in that** each one of stages 1 and 2 is carried out at a temperature from 165°C to 240°C, with a contact time from 15 minutes to 3 hours and an alcohol/feedstock mass ratio from 20/80 to 80/20.

**4.** A method as claimed in any one of claims 1 or 3, **characterized in that** the initial oil is selected from among palm oil (concrete or olein), soybean oil, palm nut oil, copra oil, babassu oil, colza oil (old or new), sunflower oil (conventional or oleic), corn oil, cotton oil, tallow and lard, peanut oil, pourgher oil, castor oil, lineseed oil and crambe oil, and all the oils obtained from sunflower or colza by genetic engineering or hybridization.

**5.** A method as claimed in any one of claims 1 or 3, **characterized in that** the initial oil is selected from waste kitchen oil, slaughterhouse oil, fish oil, seal oil, fowl fat.

**6.** A method as claimed in any one of claims 1 or 3, **characterized in that** the initial oil is selected from among oils partly modified by polymerization or oligomerization.

**7.** A method as claimed in any one of claims 1 or 3, **characterized in that** the initial oil has an acid number close to 10.

**8.** A method as claimed in any one of claims 1 or 3, **characterized in that**, the initial oil having an acid number above 10, the transesterification reaction is preceded by an esterification reaction of the free fatty acids present by means of the same alcohol as the alcohol used in the transesterification method in the presence of a strong acid selected from among sulfuric acid and soluble or supported sulfonic acids.

**9.** A method as claimed in any one of claims 1 or 3, **characterized in that**, the initial oil having an acid number above 10, the transesterification reaction is preceded by an esterification reaction of the free fatty acids present by means of glycerin to form a total or partial glycerol ester, using a heterogeneous catalyst, at atmospheric pressure or under partial vacuum, and at a temperature ranging between 165°C and 240°C.

**10.** A method as claimed in any one of claims 1 to 9, **characterized in that** said heterogeneous catalyst used in stage 1 or said heterogeneous catalyst used in stage 2 comprises:

- a combination of at least one oxide of at least one metal $M^1$ selected from among the metals of group IIA, group IIB, group IIIA, group IIIB, group IVB, group IIIA, group IVA and group VA with alumina,
- or a combination of at least two oxides of at least one metal $M^1$ and of at least one metal $M^2$ selected from among the metals of group IIA, group IIB, group IIIA, group IIIB, group IVB, group IIIA, group IVA and group VA.

**11.** A method as claimed in claim 10, wherein said catalyst comprises a combination of at least two oxides of at least one metal $M^1$ and of at least one metal $M^2$ selected from among the metals of group IIA, group IIB, group IIIA, group IIIB, group IVB, group IIIA, group IVA and group VA in the presence of alumina.

**12.** A method as claimed in claim 10, **characterized in that** said heterogeneous catalyst comprises a combination of oxides having the general formula as follows: $(M^1O_x)_y\ (Al_2O_3)_{(1-y)}$, where $\underline{x}$ has a value from 1.2 to 2.6 and y representing the mass ratio of the two oxides has a value from 0.005 to 2.

**13.** A method as claimed in claim 10, **characterized in that** said heterogeneous catalyst comprises a combination of oxides having the general formula as follows:

$$[M^1{}_aM^2{}_b\ O_c]_y\ [(Al_2O_3)]_{(1-y)}$$

where $\underline{a}$ has a value from 0.5 to 5, $\underline{b}$ a value from 0.5 to 5, $\underline{c}$ is the number of oxygen atoms that satisfies the valence of metals $M^1$ and $M^2$ and $\underline{y}$ has a value from 0.005 to 1.

## Patentansprüche

**1.** Verfahren zur Herstellung einer Esterzusammensetzung, die Ethylester linearer Monocarbonsäuren mit 6 bis 26 Kohlenstoffatomen aus einem pflanzlichen oder tierischen, säurefreien oder säurehaltigen, nativen oder recycelten Öl umfasst, wobei das Verfahren **dadurch gekennzeichnet ist, dass** es Folgendes umfasst:

- einen Schritt 1 der Reaktion des pflanzlichen oder tierischen Öls mit Methanol in Gegenwart eines heterogenen Katalysators, der eine Kombination umfasst, die zwischen mindestens einem Metalloxid und Aluminiumoxid gebildet wird, oder eine Kombination, die zwischen mindestens zwei Metalloxiden gebildet wird, und
- einen Schritt 2 der Reaktion des aus Schritt 1 stammenden Produkts mit Ethanol in Gegenwart eines heterogenen Katalysators, der auf die gleiche Weise definiert ist wie der heterogene Katalysator, der in Schritt 1 verwendet wird.

**2.** Verfahren zur Herstellung nach Anspruch 1, wobei der Katalysator eine Kombination umfasst, die zwischen mindestens zwei Metalloxiden mit Aluminiumoxid gebildet wird.

3. Verfahren nach einem der Ansprüche 1 oder 2, **dadurch gekennzeichnet, dass** jeder der Schritte 1 und 2 bei einer Temperatur von 165 bis 240°C mit einer Kontaktdauer von 15 Minuten bis 3 Stunden und einem Massenverhältnis Alkohol/Beschickung von 20/80 bis 80/20 durchgeführt wird.

4. Verfahren nach einem der Ansprüche 1 bis 3, **dadurch gekennzeichnet, dass** das Ausgangsöl ausgewählt ist aus den (konkreten oder oleinhaltigen) Palmölen, Sojaölen, Palmkernölen, Kokosölen, Babassuölen, alten und neuen Rapsölen, herkömmlichen oder ölsäurehaltigen Sonnenblumenölen, Maisölen, Baumwollsamenölen, Talg und Schweineschmalz, Erdnussölen, Purgiernussölen, Rizinusölen, Leinölen und Krambeölen und Sonnenblumenölen oder Rapsölen, die durch gentechnische Veränderung oder durch Hybridisierung erhalten wurden.

5. Verfahren nach einem der Ansprüche 1 bis 3, **dadurch gekennzeichnet, dass** das Ausgangsöl ausgewählt ist aus den Frittierölen, den Kadaverölen, den Fischölen, den Robbenölen, den Geflügelfetten.

6. Verfahren nach einem der Ansprüche 1 bis 3, **dadurch gekennzeichnet, dass** das Ausgangsöl ausgewählt ist aus den durch Polymerisation oder Oligomerisation teilweise modifizierten Ölen.

7. Verfahren nach einem der Ansprüche 1 bis 3, **dadurch gekennzeichnet, dass** das Ausgangsöl eine Säurezahl nahe an 10 aufweist.

8. Verfahren nach einem der Ansprüche 1 bis 3, **dadurch gekennzeichnet, dass**, wobei das Ausgangsöl eine Säurezahl von mehr als 10 aufweist, der Umesterungsreaktion eine Veresterungsreaktion der vorhandenen freien Fettsäuren mittels des gleichen Alkohols vorausgeht, der auch im Verfahren zur Umesterung in Gegenwart einer starken Säure, ausgewählt aus der Schwefelsäure, den löslichen oder geträgerten Sulfonsäuren, verwendet wird.

9. Verfahren nach einem der Ansprüche 1 bis 3, **dadurch gekennzeichnet, dass**, wobei das Ausgangsöl eine Säurezahl von mehr als 10 aufweist, der Umesterungsreaktion eine Veresterungsreaktion der vorhandenen freien Fettsäuren mittels Glycerin vorausgeht, um unter Verwendung eines heterogenen Katalysators bei Atmosphärendruck oder unter Teilvakuum und bei einer Temperatur im Bereich zwischen 165 und 240 °C einen Glycerolvollester oder Glycerolpartialester zu bilden.

10. Verfahren nach einem der Ansprüche 1 bis 9, **dadurch gekennzeichnet, dass** der heterogene Katalysator, der in Schritt 1 eingesetzt wird, oder der heterogene Katalysator, der in Schritt 2 eingesetzt wird, Folgendes umfasst:

   - eine Kombination aus mindestens einem Oxid mindestens eines Metalls $M^1$, ausgewählt aus den Metallen der Gruppe IIA, der Gruppe IIB, der Gruppe IIIA, der Gruppe IIIB, der Gruppe IVB, der Gruppe IVA, der Gruppe VA mit Aluminiumoxid;
   - oder eine Kombination aus mindestens zwei Oxiden mindestens eines Metalls $M^1$ und mindestens eines Metalls $M^2$, ausgewählt aus den Metallen der Gruppe IIA, der Gruppe IIB, der Gruppe IIIA, der Gruppe IIIB, der Gruppe IVB, der Gruppe IVA, der Gruppe VA.

11. Verfahren nach Anspruch 10, wobei der Katalysator eine Kombination aus mindestens zwei Oxiden mindestens eines Metalls $M^1$ und mindestens eines Metalls $M^2$, ausgewählt aus den Metallen der Gruppe IIA, der Gruppe IIB, der Gruppe IIIA, der Gruppe IIIB, der Gruppe IVB, der Gruppe IVA, der Gruppe VA, in Gegenwart von Aluminiumoxid umfasst.

12. Verfahren nach Anspruch 10, **dadurch gekennzeichnet, dass** der heterogene Katalysator eine Kombination von Oxiden mit der folgenden allgemeinen Formel umfasst:

   $(M^1O_x)_y\ (Al_2O_3)_{(1-y)}$, wobei $\underline{x}$ einen Wert von 1,2 bis 2,6 aufweist und y, das für das Massenverhältnis der beiden Oxide steht, einen Wert von 0,005 bis 2 aufweist.

13. Verfahren nach Anspruch 10, **dadurch gekennzeichnet, dass** der heterogene Katalysator eine Kombination von Oxiden mit der folgenden allgemeinen Formel umfasst:

$$[M^1{}_aM^2{}_b\ O_c]_y\ [(Al_2O_3)]_{(1-y)}$$

   wobei $\underline{a}$ einen Wert von 0,5 bis 5, $\underline{b}$ einen Wert von 0,5 bis 5 aufweist, $\underline{c}$ die Anzahl der Sauerstoffatome ist, die der Valenz der Metalle $M^1$ und $M^2$ entspricht, und $\underline{y}$ einen Wert von 0,005 bis 1 aufweist.

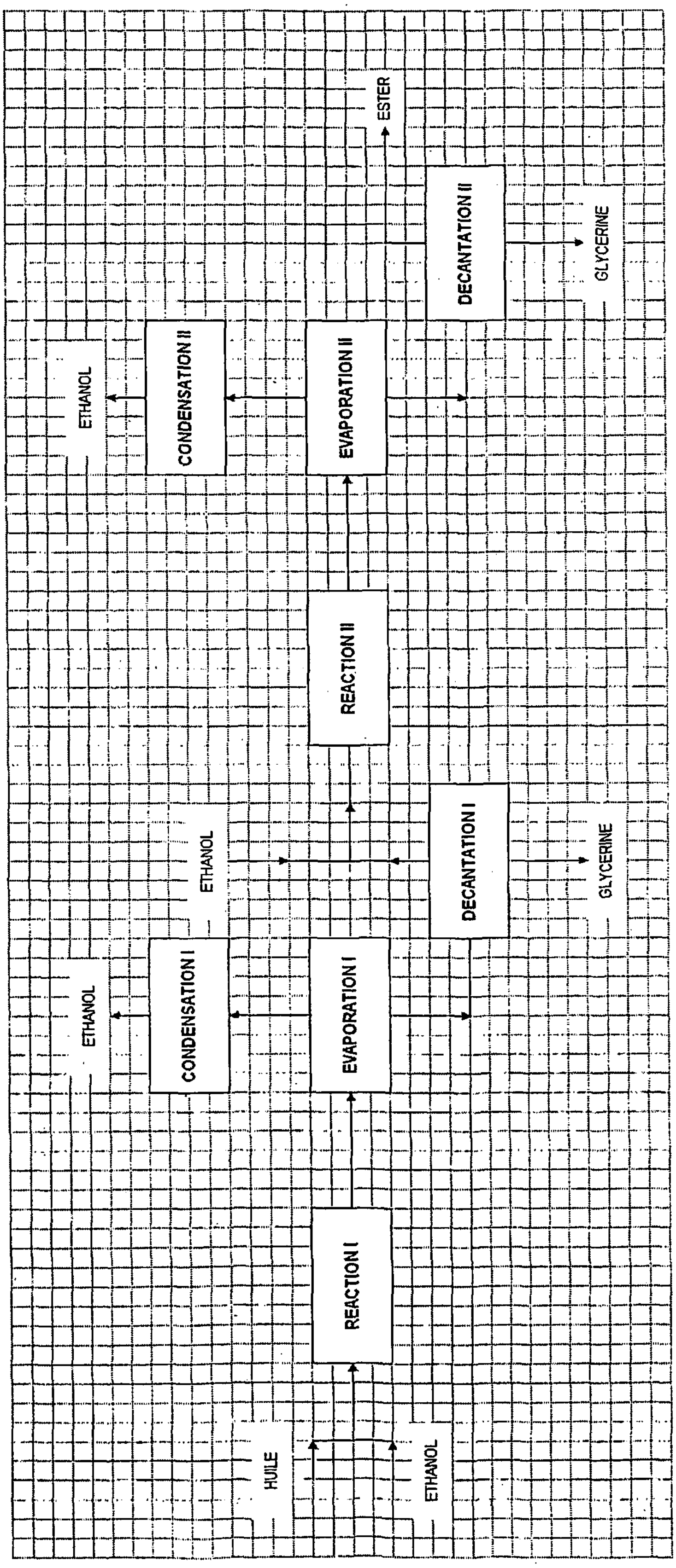
HUILE
ETHANOL
REACTION I
EVAPORATION I
CONDENSATION I
ETHANOL
DECANTATION I
GLYCERINE
ETHANOL
REACTION II
EVAPORATION II
CONDENSATION II
ETHANOL
DECANTATION II
GLYCERINE
ESTER

FIG. 1

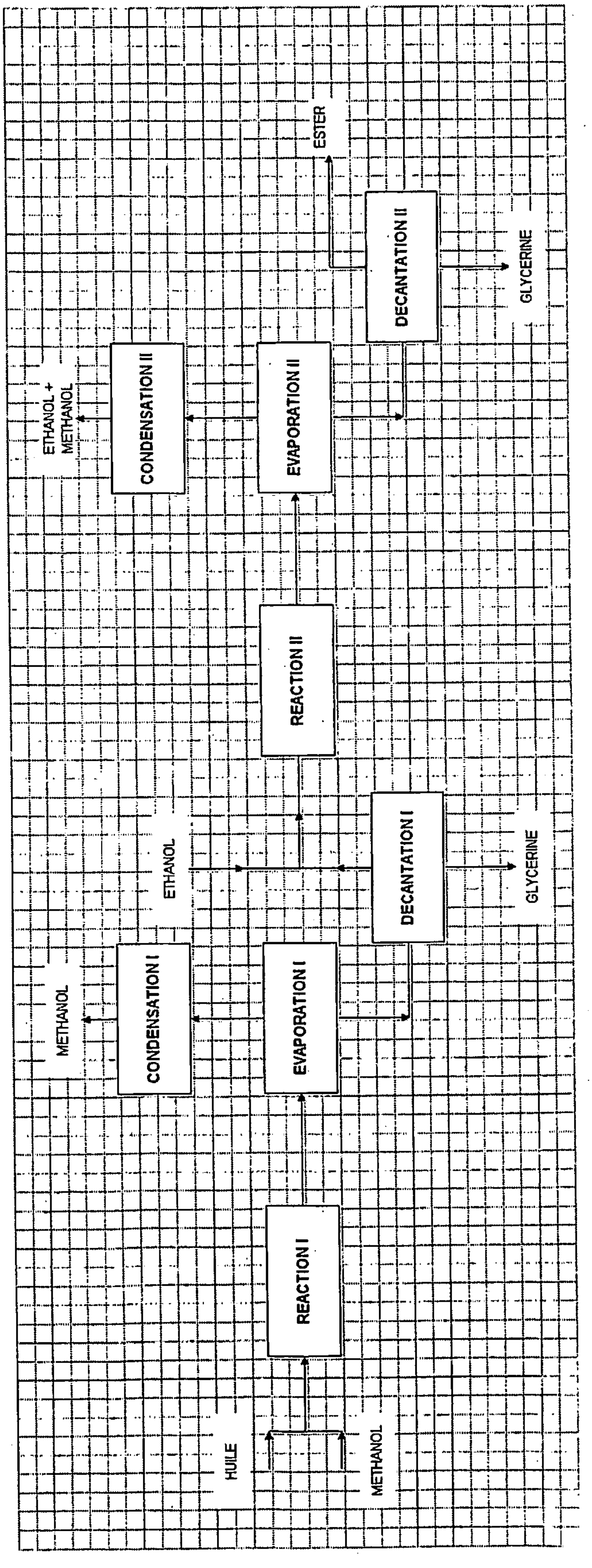
HUILE
METHANOL
REACTION I
EVAPORATION I
CONDENSATION I
METHANOL
DECANTATION I
GLYCERINE
ETHANOL
REACTION II
EVAPORATION II
CONDENSATION II
ETHANOL + METHANOL
DECANTATION II
GLYCERINE
ESTER

FIG. 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0198243 B
- GB 795573 A
- FR 2752242 B
- FR 2838433 B
- FR 2855517 A
- FR 2855518 A
- FR 2855519 A
- FR 2869612 A
- FR 2869613 A
- US 5908946 A


### Littérature non-brevet citée dans la description


- *JAOCS,* 1984, vol. 61, 343-348